# EUROPEAN PATENT SPECIFICATION

(11) **EP 1 553 950 B1**
(45) Date of publication and mention of the grant of the patent: **08.08.2007**
(21) Application number: 03751038.5
(22) Date of filing: 07.10.2003
(51) Int. Cl.: A61K 31/517, A61K 45/00

(54) **THERAPEUTIC TREATMENT**
THERAPEUTISCHE BEHANDLUNG
TRAITEMENT THERAPEUTIQUE

(30) Priority: 12.10.2002 GB 0223854
(43) Date of publication of application: 20.07.2005
(73) Proprietor: AstraZeneca AB, 151 85 (SE)
(72) Inventor: BOYLE, Francis Thomas, AstraZeneca R & D Alderley, Macclesfield, Cheshire SK10 4TG (GB); CURWEN, Jon Owen, AstraZeneca R & D Alderley, Macclesfield, Cheshire SK10 4TG (GB); GALLAGHER, Neil James, AstraZeneca, Wilmington, MA 19850-5437 (US); HANCOX, Ursula Joy, AstraZeneca R & D Alderley, Macclesfield, Cheshire SK10 4TG (GB); HUGHES, Andrew Mark, AstraZeneca R & D Alderley, Macclesfield, Cheshire SK10 4TG (GB); JOHNSTONE, Donna, AstraZeneca R & D Alderley, Macclesfield, Cheshire SK10 4TG (GB); TAYLOR, Sian Tomiko, AstraZeneca R & D Alderley, Macclesfield, Cheshire SK10 4TG (GB); TONGE, David William, AstraZeneca R & D Alderley, Macclesfield, Cheshire SK10 4TG (GB)
(86) International application number: PCT/GB2003/004347
(87) International publication number: WO 2004/035057

(56) References cited:
- WO-A-99/55683
- US-A- 5 866 568
- US-A1- 2002 107 284
- CARDUCCI M A ET AL: "ENDOTHELIN RECEPTOR ANTAGONIST, ABT-627, FOR PROSTATE CANCER: INITIAL TRIAL RESULTS" JOURNAL OF UROLOGY, BALTIMORE, MD, US, vol. 161, no. 4, SUPPL, April 1999 (1999-04), page 176 XP001037609 ISSN: 0022-5347

## Description

The present invention relates to a combination comprising an endothelin receptor antagonist ZD4054, or a pharmaceutically acceptable salt thereof, and an epidermal growth factor receptor (EGFR) tyrosine kinase inhibitor (TKI), or a pharmaceutically acceptable salt thereof. This combination is useful in a new method for the treatment or prophylaxis of cancer. The invention also relates to a pharmaceutical composition comprising such a combination and to the use thereof in the manufacture of a medicament for use in the treatment or prophylaxis of cancer, particularly prostate cancer.

Cancer affects an estimated 10 million people worldwide. This figure includes incidence, prevalence and mortality. More than 4.4 million cancer cases are reported from Asia, including 2.5 million cases from Eastern Asia, which has the highest rate of incidence in the world. By comparison, Europe has 2.8 million cases, North America 1.4 million cases, and Africa 627,000 cases.

In the UK and US, for example, more than one in three people will develop cancer at some point in their life. Cancer mortality in the U.S. is estimated to account for about 600,000 a year, about one in every four deaths, second only to heart disease in percent of all deaths, and second to accidents as a cause of death of children 1-14 years of age. The estimated cancer incidence in the U.S. is now about 1,380,000 new cases annually, exclusive of about 900,000 cases of non-melanotic (basal and squamous cell) skin cancer.

Cancer is also a major cause of morbidity in the UK with nearly 260,000 new cases (excluding non-melanoma skin cancer) registered in 1997. Cancer is a disease that affects mainly older people, with 65% of cases occurring in those over 65. Since the average life expectancy in the UK has almost doubled since the mid nineteenth century, the population at risk of cancer has grown. Death rates from other causes of death, such as heart disease, have fallen in recent years while deaths from cancer have remained relatively stable. The result is that 1 in 3 people will be diagnosed with cancer during their lifetime and 1 in 4 people will die from cancer. In people under the age of 75, deaths from cancer outnumber deaths from diseases of the circulatory system, including ischaemic heart disease and stroke. In 2000, there were 151,200 deaths from cancer. Over one fifth (22 per cent) of these were from lung cancer, and a quarter (26 per cent) from cancers of the large bowel, breast and prostate.

Worldwide, the incidence and mortality rates of certain types of cancer (of stomach, breast, prostate, skin, and so on) have wide geographical differences which are attributed to racial, cultural, and especially environmental influences. There are over 200 different types of cancer but the four major types, lung, breast, prostate and colorectal, account for over half of all cases diagnosed in the UK and US. Prostate cancer is the fourth most common malignancy among men worldwide, with an estimated 400,000 new cases diagnosed annually, accounting for 3.9 percent of all new cancer cases.

Current options for treating cancers include surgical resection, external beam radiation therapy and / or systemic chemotherapy. These are partially successful in some forms of cancer, but are not successful in others. There is a clear need for new therapeutic treatments.

Recently, endothelin A receptor antagonists have been identified as potentially of value in the treatment of cancer (Cancer Research, 56, 663-668, February 15^{th}, 1996 and Nature Medicine, Volume 1, Number 9, September 1999,944-949).

The endothelins are a family of endogenous 21 amino acid peptides comprising three isoforms, endothelin-1 (ET-1), endothelin-2 and endothelin-3. The endothelins are formed by cleavage of the Trp²¹-Val²² bond of their corresponding proendothelins by an endothelin converting enzyme. The endothelins are among the most potent vasoconstrictors known. They exhibit a wide range of other activities including stimulation of cell proliferation and mitogenesis, inhibition of apoptosis, extravasation and chemotaxis, and also interact with a number of other vasoactive agents.

The endothelins are released from a range of tissue and cell sources including vascular endothelium, vascular smooth muscle, kidney, liver, uterus, airways, intestine and leukocytes. Release can be stimulated by hypoxia, shear stress, physical injury and a wide range of hormones and cytokines. Elevated endothelin levels have been found in a number of disease states in man including cancers.

In recent years it has been discovered that certain growth factor tyrosine kinase enzymes are important in the transmission of biochemical signals which initiate cell replication. They are large proteins which span the cell membrane and possess an extracellular binding domain for growth factors such as epidermal growth factor (EGF) and an intracellular portion which functions as a kinase to phosphorylate tyrosine amino acids in proteins and hence to influence cell proliferation.

Various classes of receptor tyrosine kinases are known (Wilks, Advances in Cancer Research, 1993, 60, 43-73) based on families of growth factors which bind to different receptor tyrosine kinases. The classification includes Class I receptor tyrosine kinases comprising the EGF family of receptor tyrosine kinases such as the EGF, TGFα, NEU, erbB, Xmrk, HER and let23 receptors, Class II receptor tyrosine kinases comprising the insulin family of receptor tyrosine kinases such as the insulin and IGF1 receptors and insulin-related receptor (IRR) and Class III receptor tyrosine kinases comprising the platelet-derived growth factor (PDGF) family of receptor tyrosine kinases such as the PDGFα, PDGFβ and colony-stimulating factor 1 (CSF1) receptors.

It is known that Class I kinases such as the EGF family of receptor tyrosine kinases are frequently present in common human epithelial cancers such as cancer of the prostate (Visakorpi et al., Histochem. J., 1992, 24, 481). Accordingly it has been recognised that an inhibitor of receptor tyrosine kinases should be of value as a selective inhibitor of the growth of certain carcinomas.

It has been previously demonstrated that stimulation of rat-1 fibroblast cells with endothelin-1 resulted in transactivation of the EGFR *in vitro* (Daub, H et al., Nature, 1996, 379:557). Since both the endothelin and EGFR systems play a role in carcinogenesis the present inventors investigated the potential for the combined use of endothelin antagonists and EGFR TKIs for the treatment of cancer. The present inventors have unexpectedly found that the combination use of particular endothelin receptor antagonists, or pharmaceutically acceptable salts thereof, and particular EGFR TKIs, or pharmaceutically acceptable salts thereof, can have a synergistic and or additive effect in the treatment of cancer.

Therefore according to the present invention, there is provided a combination, comprising an endothelin receptor antagonist ZD4054; or a pharmaceutically acceptable salt thereof, and an EGFR TKI, or a pharmaceutically acceptable salt thereof.

Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the benefit of the synergistic and / or additive effect of the combination.

In one aspect, where a compound or a pharmaceutically acceptable salt thereof, is referred to this refers to the compound only. In another aspect this refers to a pharmaceutically acceptable salt of the compound.

Where cancer is referred to, particularly it refers to oesophageal cancer, myeloma, hepatocellular, pancreatic, cervical cancer, ewings tumour, neuroblastoma, kaposis sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, brain cancer, renal cancer, lymphoma and leukaemia. More particularly it refers to prostate cancer. In addition, more particularly it refers to SCLC, NSCLC, colorectal cancer, ovarian cancer and / or breast cancer. More particularly it refers to SCLC. More particularly it refers to NSCLC. More particularly it refers to colorectal cancer. In addition, more particularly it refers to ovarian cancer. More particularly it refers to breast cancer. More particularly it refers to renal cancer. Furthermore, more particularly it refers to bladder cancer, oesophageal cancer, gastric cancer, melanoma, cervical cancer and / or renal cancer. In addition it refers to endometrial, liver, stomach, thyroid, rectal and / or brain cancer. In another aspect of the invention, the cancer is not melanoma. In another embodiment of the invention, particularly the cancer is in a metastatic state, and more particularly the cancer produces metastases to the bone. In a further embodiment of the invention, particularly the cancer is in a metastatic state, and more particularly the cancer produces skin metastases. In a further embodiment of the invention, particularly the cancer is in a metastatic state, and more particularly the cancer produces lymphatic metastases. In a further embodiment of the invention, the cancer is in a non-metastatic state.

Where the treatment of cancer is referred to particularly this is the treatment of cancerous tumours expressing both endothelin A and EGFR. This treatment is in terms of one or more of the extent of the response, the response rate, the time to disease progression and the survival rate. It is further expected that the combination use of the particular endothelin receptor antagonists, or pharmaceutically acceptable salts thereof, and particular EGFR TKIs, or pharmaceutically acceptable salts thereof, will have a beneficial effect in preventing the onset of cancer in warm-blooded animals, such as man.

The compounds, or a pharmaceutically acceptable salt thereof, possessing endothelin receptor antagonist activity is described in WO 96/40681, WO 95/26716, WO 95/26360, WO95/26957, WO 95/33748, WO 95/33752, WO 95/35107, WO 96/04905, WO 96/06095, WO 96/07653, WO 96/08483, WO 96/08486, WO 96/08487, WO 96/09818, WO 96/11914, WO 96/11927, WO 96/12706, WO 96/15109, WO 96/19455, WO 96/19459, WO 96/22978, WO 96/23773, WO 96/30358, WO 96/31492, WO 96/33170, WO 96/33190, WO96/40681, WO 97/30045, WO 98/09953, WO 95/08550, WO 98/49162, WO 99/06397, WO 01/49685, WO 02/64573, EP 436189, EP 496452, EP 510526, EP 526708, EP 552417, EP 555537, EP 601386, EP 617001, EP 628569, BP 633259, EP 658548, EP 682016, EP 713875, EP 702012, EP 733626, EP 743307, EP 749964, GB2266890, GB 2275926, GB 2276383, GB 2277446, GB 2295616, DE 4341663, JP 6256261, JP 6122625, JP 7330622, JP 7133254, JP 8059635, JP 7316188, and JP 7258098 and the receptor antagonists described therein, particularly those described in claim 1 and the named examples, of the above patents and applications, are incorporated herein by reference.

Additional suitable compounds, or a pharmaceutically acceptable salt thereof, possessing endothelin receptor antagonist activity include those described in the J Med Chem papers 1996, 39, 2123-2128; **1997,** *40,* 3, 322-330; **2001,** *44*, 1211-1216; **2001,** *44*, 3978-3984 and the endothelin receptor antagonists described therein are also incorporated herein by reference.

Further suitable compounds, or a pharmaceutically acceptable salt thereof, possessing endothelin receptor antagonist activity include A-127722, atrasentan (ABT-627), BQ-123, BQ-788, BMS 182874, feloprentan, BSF 420627, FR139317, IPI-950, L-749,329, L-754,142, LU 110896, LU 110897, PD 156707, PD 155080, Ro 46-2005, bosentan (Ro 47-0203), SB 217242, SB 209670, TAK-044, YM598, sitaxsentan (TBC11251), ambrisentan, tezosentan, darusentan, *N*-[[2'-[[(4,5-dimethyl-3-isoxazolyl)amino]sulphonyl]-4-(2-oxazolyl)[1,1'-biphenyl]-2-yl]methyl]-*N*,3,3-trimethylbutanamide, ZD1611 and *N*-(3-methoxy-5-methylpyrazin-2-yl)-2-(4-[1,3,4-oxadiazol-2-yl]phenyl)pyridine-3-sulphonamide (ZD4054).

Further suitable compounds, or a pharmaceutically acceptable salt thereof, possessing endothelin receptor antagonist activity include A-104029, A-158112, A-182086, A-192621, A-201661, A-203719, A-206377, A-207508, A-308165, ABT-306552, ABT-546, BE-18257B, BQ-017, BQ-145, BQ-238, BQ-518, BQ-928, J-104121, J-104132, J-112287, BSF-461314, BMS-187308, BMS-193884, cdonentan, IRL-1543, IRL-1722, IRL-1841, TBC-10662, TBC-11040, TBC-11299, TBC-3711, Ro-48-5694, Ro-61790C, VML-588, FR-901367, FR-901533, Ro-46-8443, IPI-616, LU-127043, K-8794, RES 1214-1, RES-1149-1, RES-701-1, RES-701-2, BQ-153, BQ-485, BQ-610, L-744453, L-746072,

Suitable compounds, or a pharmaceutically acceptable salt thereof, possessing EGFR TKI activity include those described in EP 0566226, EP 0787722, WO 96/30347, WO 96/33980, WO 97/02266, WO 97/30034, WO 97/38983, WO 98/50038, WO 99/09016, WO 99/24037, WO 99/55683, Nature Medicine, 2000, 6, 1024-1028 and US 6,002,008 and these EGFR TKIs, particularly those of claim 1 and the named examples of these patents.

Particular classes of EGFR TKIs are the quinazolines, or a pharmaceutically acceptable salt thereof.

Particular compounds, or pharmaceutically acceptable salts thereof possessing EGFR TKI activity include:
*N*-(3-chloro-4-fluorophenyl)-7-methoxy-6-(3-morpholinopropoxy)quinazolin-4-amine (ZD1839);
*N*-(3-ethynylphenyl)-6,7-bis(2-methoxyethoxy)quinazolin-4-amine, or a pharmaceutically-acceptable salt thereof (linked to the code numbers CP 358774 and OSI-774 (the monomethanesulphonate salt));
6-acrylamido-*N*-(3-chloro-4-fluorophenyl)-7-(3-morpholinopropoxy)quinazolin-4-amine (linked to the code numbers PD 183805 and CI 1033);
4-[(1R)-1-phenylethylamino]-6-(4-hydroxyphenyl)-7H-pyrrolo[2,3-*d*]pyrimidine (linked to the code numbers PKI-166, CGP 75166 and CGP 59326);
*N*-[4-(3-bromoanilino)quinazolin-6-yl]but-2-ynamide (linked to the code numbers CL-387785 and EKB-785); and
4-(3-chloro-4-fluoroanilino)-3-cyano-6-(4-dimethylaminobut-2(E)-enamido)-7-ethoxyquinoline (EKB-569).

Further particular compounds, or pharmaceutically acceptable salts thereof, possessing EGFR TKI activity include ZD1839, CP 358774, CI 1033, PKI-166, CL-387785 and EKB-569. Particularly the compound, or a pharmaceutically acceptable salt thereof, possessing EGFR TKI activity is ZD1839 or CP 358774. More particularly the compound, or a pharmaceutically acceptable salt thereof, possessing EGFR TKI activity is ZD1839. Further particular compounds, or pharmaceutically acceptable salts thereof, possessing EGFR TKI activity include BE-23372M, BE-23372M derivatives Banyu, BIBX-1382, BBR-1611, naamidine A, AS-23, DAB-720, ADL-681, CGP-52411, CGP-60261, CGP-62706 series, cetuximab, PKI-166, CP-292597, erlotinib, PD-0158780, hbEGF-toxin, Prizm, RG-13022, RG-14620, RG-50875, AG-1478, VRCTC-310, SU-5271, Particular combinations of the present invention include:
- ZD4054, or a pharmaceutically acceptable salt thereof, and ZD1839, or a pharmaceutically acceptable salt thereof;
- ZD4054, or a pharmaceutically acceptable salt thereof, and CP 358774, or a pharmaceutically acceptable salt thereof;

In any of the combinations described herein, including the use of these combinations, kits or formulations containing them etc., an additional compound or compounds (see below) can optionally be present. The combination of an endothelin receptor antagonist, or a pharmaceutically acceptable salt thereof, and an EGFR TKI, or a pharmaceutically acceptable salt thereof, can optionally be administered in further combination with:
i) an LHRH analogue; and / or
ii) a bisphosphonate.

Herein where the term "LHRH analogue" is used it is to be understood that this refers to any chemical compound, or a pharmaceutically acceptable salt thereof, including small molecules and peptides, which acts as an agonist or antagonist at the LHRH receptor, whether by an interaction with the LHRH binding site or by an allosteric mechanism, i.e. acts at a position on the LHRH receptor different to the LHRH binding site. In one aspect of the invention an "LHRH analogue" refers to an LHRH antagonist or a pharmaceutically acceptable salt thereof. In one aspect of the invention an "LHRH analogue" refers to an LHRH agonist or a pharmaceutically acceptable salt thereof. In a further aspect of the invention an "LHRH analogue" refers to a combination of an LHRH antagonist or a pharmaceutically acceptable salt thereof and an LHRH agonist or a pharmaceutically acceptable salt thereof.

Particular compounds, or pharmaceutically acceptable salts thereof possessing LHRH analogue activity include Azaline B, A-198401, A-75998, A-76154, A-84861, abarelix, AN-152, AN-207, Antide, avorelin, cetrorelix, D-21775, D-23487, D-26344, D-63153, D-85108, degarelix, deslorelin, detirelix, FE 200486, ganirelix, gonadimmune, goserelin, histrelin, leuprolide, leuprorelin, metarelin, nafarelin, NBI-42902 (Neurocrine), Org-30850, PH-45 (Pherin Corp), PTL-03001, ramorelix, RWJ-47428-021, SPD-424, surfagon, T-66 (Matrix Therapeutics Ltd), TAK-013, TAK-810, teverelix, triptorelin acetate, triptorelin pamoate, vomeropherin or ZK-157348.

Particular LHRH analogues are peptides or peptide derivatives.

Examples of particular LHRH agonists include, but are not limited to;
i) buserelin (US Patent 4 024 248)
   (pyr)Glu-His-Trp-Ser-Tyr-D-Ser(Bu^{t})⁶-Leu-Arg-Pro-NHCH₂CH₃
ii) triptorelin (US Patent 4 010 125)
   (pyr)Glu-His-Trp-Ser-Tyr-Trp-Leu-Arg-Pro-Gly-NH₂
iii) leuprorelin (Us Patent 4 005 063)
   (pyr)Glu-His-Trp-Ser-Tyr-D-Leu-Leu-Arg-Pro-NHCH₂CH₃
iv) goserelin (US Patent 4 100 274)
   (pyr)Glu-His-Trp-Ser-Tyr- D-Ser(Bu^{t})⁶-Leu-Arg-Pro-(Azygly)NH₂
v) deslorelin (US Patent 4 659 695)
   (pyr)Glu-His-Trp-Ser-Tyr-D-Trp-Leu-Arg-Pro-NH-CH₂-CH₂-NH₂
vi) histerelin (US Patent 4 244 946)
   (pyr)Glu-His-Trp- Ser-Tyr-D-His(Bzl)-Leu-Arg-Pro-NH-CH₂-CH₃
vii) avorelin (US 5 668 254)
   (pyr)Glu-His-Trp-Ser-Tyr-D-Trp(2-Me)-Leu-Arg-Pro-NH-CH₂-CH₃
viii) nafarelin (US Patent 4 234 571)
   (pyr)Glu-His-Trp-Ser-Tyr-D-Nal(2)-Leu-Arg-Pro-NH-CH₂-CH₃; lutrelin, cystorelin, gonadorelin or detirelix.

Particularly the LHRH agonist is selected from leuprorelin, buserelin, triptorelin and goserelin. More particularly the LHRH agonist is goserelin.

Examples of suitable LHRH antagonists include, but are not limited to, antide, abarelix, antarelix, cetrorelix, azaline, ganirelix and those disclosed in US Patents 5 470 947 (Folkers); 5 413 990 and 5 300 492 (Haviv); 5 371 070 (Koerber); 5 296 468 (Hoeger); 5 171 635 (Janaky); 5 003 011 and 4 431635 (Coy); 4 992 421 (De); 4 801 577 (Nestor); and 4 851 385, 4 689 396 and 5 843 901 (Roeske).

Further examples of suitable LHRH antagonists include, but are not limited to the compounds described in WO 02/066477, WO 02066478, WO 02/066459, WO 02/092565, PCT/GB03/003603 and PCT/GB03/003606, and the compounds described in these applications, particularly the compounds of claim 1 and the named examples.

A "bisphosphonate" is a compound, or a pharmaceutically acceptable salt thereof, capable of regulating metal cations content (especially calcium content) in humans and is a compound containing two carbon phosphorous bonds. For further explanation of the term "bisphosphonate" the readers attention is drawn to Endocrine Reviews, 1998, 19(1): 80-100, the content of which is incorporated herein by reference.

Particular bisphosphonates for use in the present invention are selected from tiludronic acid, ibandronic acid, incadronic acid, risedronic acid, zoledronic acid, clodronic acid, neridronic acid, pamidronic acid, alendronic acid, minodronic acid, olpadronic acid, TRK 530, CGP 47072, calcium clodronate or EB 1053. Further particular bisphosphonates for use in the present invention are selected from etidronic acid, PNU-91638, NE-21650, NE-58025, NE-10790 or NE-10446.

Suitable pharmaceutically-acceptable salts include, for example, salts with alkali metal (such as sodium, potassium or lithium), alkaline earth metals (such as calcium or magnesium), ammonium salts, and salts with organic bases affording physiologically acceptable cations, such as salts with methylamine, dimethylamine, trimethylamine, piperidine and morpholine. In addition, for those compounds which are sufficiently basic, suitable pharmaceutically-acceptable salts include, pharmaceutically-acceptable acid-addition salts with hydrogen halides, sulphuric acid, phosphoric acid and with organic acids such as citric acid, maleic acid, methanesulphonic acid and p-toluenesulphonic acid. Alternatively, the compounds may exist in zwitterionic form.

The therapeutic effect (for example effects on cell proliferation and / or the effect on cell survival or induction of an apoptotic response) of the combination may be tested *in vitro* by the application of an endothelin receptor antagonists and a EGFR TKI to human carcinoma cell cultures expressing both endothelin A and EGFR.

Therefore according to the present invention, there is provided a combination, comprising an endothelin receptor antagonist, or a pharmaceutically acceptable salt thereof, and an EGFR TKI, or a pharmaceutically acceptable salt thereof for use as a medicament.

According to a further aspect of the invention there is provided a pharmaceutical composition which comprises an endothelin receptor antagonist, or a pharmaceutically acceptable salt thereof, and an EGFR TKI, or a pharmaceutically acceptable salt thereof, in association with a pharmaceutically acceptable diluent or carrier.

According to a further aspect of the invention there is provided a pharmaceutical composition which comprises an endothelin receptor antagonist, or a pharmaceutically acceptable salt thereof, in association with a pharmaceutically acceptable diluent or carrier, in combination with a pharmaceutical composition which comprises an EGFR TKI, or a pharmaceutically acceptable salt thereof, in association with a pharmaceutically acceptable diluent or carrier.

Therefore according to the present invention, there is provided a method of treating cancer, in a warm-blooded animal, such as man, in need of such treatment which comprises administering to said animal an effective amount of an endothelin receptor antagonist, or a pharmaceutically acceptable salt thereof in combination with an effective amount of an EGFR TKI, or a pharmaceutically acceptable salt thereof.

For the avoidance of doubt, where the treatment of cancer is indicated, it is to be understood that this also refers to the prevention of metastases and the treatment of metastases, i.e. cancer spread. Therefore the combination of the present invention could be used to treat a patient who has no metastases to stop them occurring, or to lengthen the time period before they occur, and to a patient who already has metastases to treat the metastases themselves. Furthermore the treatment of cancer also refers to treatment of an established primary tumour or tumours and developing primary tumour or tumours. In one aspect of the invention the treatment of cancer relates to the prevention of metastases. In another aspect of the invention the treatment of cancer relates to the treatment of metastases. In another aspect of the invention the treatment of cancer relates to treatment of an established primary tumour or tumours or developing primary tumour or tumours. Herein, the treatment of cancer also refers to the prevention of cancer *per se.*

In addition the treatment of cancer also refers to the production of an anti-angiogenic effect in a warm blooded animal.

According to a further aspect of the present invention there is provided a kit comprising an endothelin receptor antagonist ZD4054, or a pharmaceutically acceptable salt thereof, and an EGFR TKI, or a pharmaceutically acceptable salt thereof; optionally with instructions for use.

According to a further aspect of the present invention there is provided a kit comprising:
a) an endothelin receptor antagonist, or a pharmaceutically acceptable salt thereof, in a first unit dosage form;
b) an EGFR TKI, or a pharmaceutically acceptable salt thereof; in a second unit dosage form; and
c) container means for containing said first and second dosage forms; and optionally
d) with instructions for use.

According to a further aspect of the present invention there is provided a kit comprising:
a) an endothelin receptor antagonist, or a pharmaceutically acceptable salt thereof, together with a pharmaceutically acceptable diluent or carrier, in a first unit dosage form;
b) an EGFR TKI, or a pharmaceutically acceptable salt thereof, in a second unit dosage form; and
c) container means for containing said first and second dosage forms; and optionally
d) with instructions for use.

According to a further aspect of the invention there is provided a pharmaceutical composition which comprises an endothelin receptor antagonist, or a pharmaceutically acceptable salt thereof, and an EGFR TKI, or a pharmaceutically acceptable salt thereof, in association with a pharmaceutically acceptable diluent or carrier for use in the treatment of cancer.

According to a further aspect of the invention there is provided a pharmaceutical composition which comprises an endothelin receptor antagonist ZD4054 or a pharmaceutically acceptable salt thereof, in association with a pharmaceutically acceptable diluent or carrier, in combination with a pharmaceutical composition which comprises an EGFR TKI, or a pharmaceutically acceptable salt thereof, in association with a pharmaceutically acceptable diluent or carrier for use in the treatment of cancer.

The pharmaceutical compositions may be in a form suitable for oral administration, for example as a tablet or capsule, for parenteral injection (including intravenous, subcutaneous, intramuscular, intravascular or infusion) as a sterile solution, suspension or emulsion, for topical administration as an ointment or cream or for rectal administration as a suppository. In general the above compositions may be prepared in a conventional manner using conventional excipients.

For example ZD4054 can be formulated as a tablet using the following excipients:
ZD4054;
Lactose monohydrate (filler);
Croscarmellose sodium (disintegrant);
Povidone (binder);
Magnesium stearate (lubricant);
Hypromellose (film coat component);
Polyethylene glycol 300 (film coat component); and
Titanium dioxide (film coat component).

According to a further aspect of the present invention there is provided a kit comprising an endothelin receptor antagonist, or a pharmaceutically acceptable salt thereof, and an EGFR TKI, or a pharmaceutically acceptable salt thereof; optionally with instructions for use; for use in the treatment of cancer.

According to a further aspect of the present invention there is provided a kit comprising:
a) an endothelin receptor antagonist, or a pharmaceutically acceptable salt thereof, in a first unit dosage form;
b) an EGFR TKI, or a pharmaceutically acceptable salt thereof; in a second unit dosage form; and
c) container means for containing said first and second dosage forms; and optionally
d) with instructions for use;
for use in the treatment of cancer.

According to a further aspect of the present invention there is provided a kit comprising:
a) an endothelin receptor antagonist ZD4054, or a pharmaceutically acceptable salt thereof, together with a pharmaceutically acceptable diluent or carrier, in a first unit dosage form;
b) an EGFR TKI, or a pharmaceutically acceptable salt thereof, in a second unit dosage form; and
c) container means for containing said first and second dosage forms; and optionally
d) with instructions for use; for use in the treatment of cancer.

According to another feature of the invention there is provided the use of an endothelin receptor antagonist ZD4054, or a pharmaceutically acceptable salt thereof, in combination with an EGFR TKI, or a pharmaceutically acceptable salt thereof, in the manufacture of a medicament for use in the treatment of cancer, in a warm-blooded animal, such as man.

According to another feature of the invention there is provided the use of an endothelin receptor antagonist, or a pharmaceutically acceptable salt thereof, in combination with an EGFR TKI, or a pharmaceutically acceptable salt thereof, in the treatment of cancer, in a warm-blooded animal, such as man.

According to a further aspect of the present invention there is provided a combination comprising an endothelin receptor antagonist ZD4054, or a pharmaceutically acceptable salt thereof, and an EGFR TKI, or a pharmaceutically acceptable salt thereof, for use in the treatment of cancer.

According to a further aspect of the present invention there is provided a combination treatment comprising the administration of an effective amount of an endothelin receptor antagonist, or a pharmaceutically acceptable salt thereof, optionally together with a pharmaceutically acceptable diluent or carrier, in combination with an effective amount of an EGFR TKI, or a pharmaceutically acceptable salt thereof, optionally together with a pharmaceutically acceptable diluent or carrier to a warm-blooded animal, such as man in need of such therapeutic treatment for use in the treatment of cancer.

The endothelin receptor antagonist, or a pharmaceutically acceptable salt thereof, will normally be administered to a warm-blooded animal at a unit dose of 1g or less daily but more than 2.5mg and this would be expected to provide a therapeutically-effective dose. However the daily dose will necessarily be varied depending upon the host treated, the particular route of administration, and the severity of the illness being treated. Accordingly the optimum dosage may be determined by the practitioner who is treating any particular patient. Particularly the endothelin antagonist could be administered to a warm-blooded animal, at a unit dose of less than 250 mg per day. In another aspect of the invention, the endothelin antagonist could be administered to a warm-blooded animal, at a unit dose of less than 130 mg per day. In a further aspect of the invention, the endothelin antagonist could be administered to a warm-blooded animal, at a unit dose of less than 50 mg per day.

The EGFR TKI, or pharmaceutically acceptable salt thereof, will normally be administered to a warm-blooded animal at a unit dose, for example, from about 20 mg to 1 g of active ingredient. When the EGFR TKI is ZD1839, a conventional tablet formulation may be used for oral administration containing 50 mg, 100 mg, 250 mg or 500 mg of active ingredient. Conveniently the daily oral dose of ZD1839 is above 150 mg, for example, in the range 150 to 750 mg, preferably in the range 200 to 500 mg. For a single dosage form, the active ingredients may be compounded with an appropriate and convenient amount of excipients which may vary from about 5 to about 98 percent by weight of the total composition. Dosage unit forms will generally contain about 20 mg to about 500 mg of each active ingredient. However the daily dose will necessarily be varied depending upon the host treated, the particular route of administration, and the severity of the illness being treated. Accordingly the optimum dosage may be determined by the practitioner who is treating any particular patient.

The dosage of each of the two drugs and their proportions have to be composed so that the best possible treatment effects, as defined by national and international guidelines (which are periodically reviewed and re-defined), will be met.

### Legends to Figures

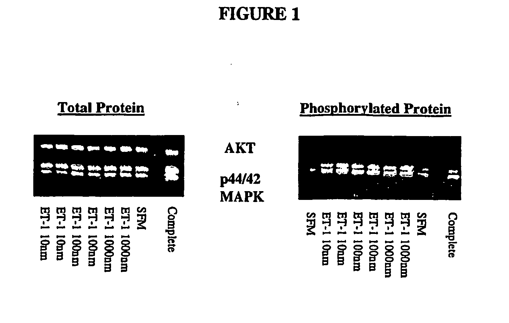

Figure 1: This is a Western Blot demonstrating that ET-1 stimulates MC-3T3. E1/J1 p44/42 MAPK phosphorylation

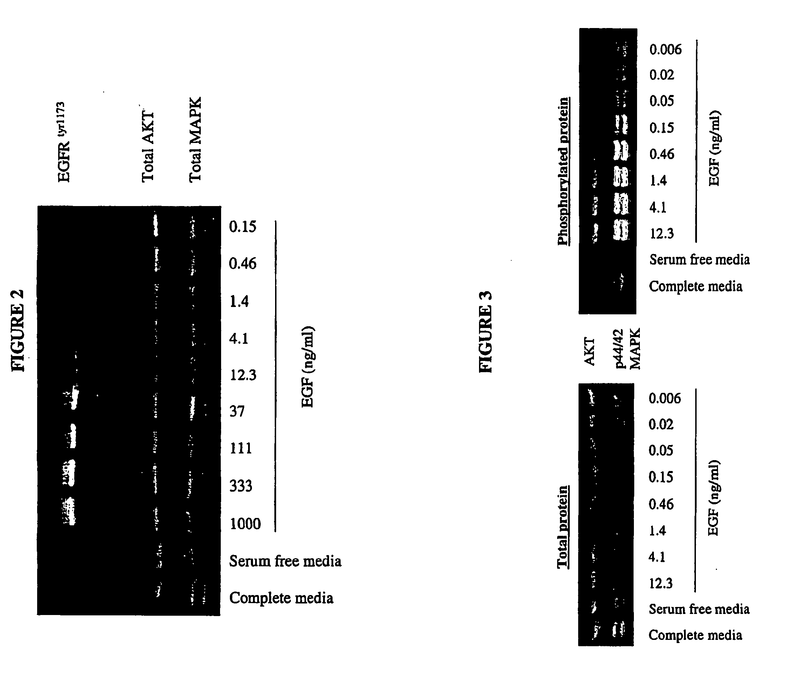

Figure 2: This is a Western Blot demonstrating that EGF stimulates MC-3T3. E1/J1 EGFR^{tyr1173} phosphorylation
Figure 3: This is a Western Blot demonstrating that EGF stimulates MC-3T3. E1/J1 AKT and p44/42 MAPK phosphorylation.

### Experimental

The following study was carried out to demonstrate the involvement of MAPK in both ET-1 and EGF osteoblastic signalling pathways

### Methodology

The MC3T3. E1/J1 cell line was isolated from a parental cell line, MC3T3-E1 (available from Invitrogen), which had in turn been derived from newborn C57BL/6 mouse calvaria. The MC3T3 E1/J1 line is described as an osteoblastic line. Care is taken in culture not to use a medium with a pH greater than 7.5 or to allow the cells to become confluent, as this causes a de-differentiation to form a fibroblastic phenotype. This reversion to a fibroblastic phenotype is a common feature of osteoblastic cell cultures.

MC3T3.E1/J1 cells were routinely maintained in complete media (α-modified minimum essential medium eagle + 10% foetal calf serum + 1% glutamine + 10% M1). MC3T3.E1/J1 cells were plated at 1.7x10⁴ cells/well (24 well plates) in complete media and incubated at 37°C in 5% CO₂ for 48 hours. Cells were washed twice in phosphate buffered saline and re-incubated for approximately 17 hours in serum starvation media (α-modified minimum essential medium eagle + 1% glutamine).

Cells were then stimulated with growth factor (ET-1 or EGF) for 3 minutes. All media was then removed and the cells lysed in 2x laemli buffer. Following electrophoresis and western blotting, membranes were probed with phospho-p44/42 mitogen activated proteine kinase (MAPK)^{(Thr20/204)}, phosphor-AKT^{(Ser 473)} and phospho-EGF Receptor^{(tyr 1173)} specific antibodies.

### Results

See Figures attached

### Conclusion

We have shown that EGF stimulates the same proliferative and survival pathways in the osteoblast as ET-1. MAPK has been shown to be involved in stimulating proliferation of the osteoblast, as-well as stimulating the production of growth factors which aid tumour cell survival in bone ^{(1,2)}. Endothelin antagonists are effective inhibitors of ET-1 mediated activation of MAPK in the osteoblast. Therefore, an endothelin antagonist and EGFR TKI combination therapy should have a beneficial effect in preventing pathological bone density changes.
¹ Kawamura et. al. (1999) "Involvement of p42/p44 MAP kinase in endothelin-1-induced interleukin-6 synthesis in osteoblast-like cells" Bone 24: 315-320
² Gonzalez, E et. al. (1996). "Retinoids Modulate the effect of PTH and Calcitriol on EGF Receptor Expression in UMR 106-01 Cells." Calcif Tissue Int 58 (6): 429-434.

## Claims

1. A combination, comprising *N*-(3-methoxy-5-methylpyrazin-2-yl)-2-(4-[1,3,4-oxadiazol-2-yl]phenyl)pyridine-3-sulphonamide (ZD4054), or a pharmaceutically acceptable salt thereof, and an EGFR TKI, or a pharmaceutically acceptable salt thereof.

2. A combination according to claim 1 wherein the EGFR TKI is selected from:
*N*-(3-chloro-4-fluorophenyl)-7-methoxy-6-(3-morpholinopropoxy)quinazolin-4-amine (ZD1839);
*N*-(3-ethynylphenyl)-6,7-bis(2-methoxyethoxy)quinazolin-4-amine, or a pharmaceutically-acceptable salt thereof (linked to the code numbers CP 358774 and OSI-774 (the monomethanesulphonate salt));
6-acrylamido-*N*-(3-chloro-4-fluorophenyl)-7-(3-morpholinopropoxy)quinazolin-4-amine (linked to the code numbers PD 183805 and CI 1033);
4-[(1R)-1-phenylethylamino]-6-(4-hydroxyphenyl)-7H-pyrrolo[2,3-*d*]pyrimidine (linked to the code numbers PKI-166, CGP 75166 and CGP 59326);
*N*-[4-(3-bromoanilino)quinazolin-6-yl]but-2-ynamide (linked to the code numbers CL-387785 and EKB-785); and
4-(3-chloro-4-fluoroanilino)-3-cyano-6-(4-dimethylaminobut-2(E)-enamido)-7-ethoxyquinoline (EKB-569);
or a pharmaceutically acceptable salt thereof.

3. A combination according to claim 1 or 2 wherein the EGFR TKI is selected from ZD1839, or a pharmaceutically acceptable salt thereof.

4. A combination according to any one of claims 1-3 for use as a medicament.

5. A pharmaceutical composition comprising a combination according to any one of claims 1-3, in association with a pharmaceutically acceptable diluent or carrier.

6. The use of a combination according to any one of claims 1-3, in the manufacture of a medicament for use in the treatment of cancer, in a warm-blooded animal, such as man.

7. The use according to claim 6 wherein the cancer is oesophageal cancer, myeloma, hepatocellular, pancreatic, cervical cancer, ewings tumour, neuroblastoma, kaposis sarcoma, ovarian cancer, breast cancer, colorectal cancer, prostate cancer, bladder cancer, melanoma, lung cancer - non small cell lung cancer (NSCLC), and small cell lung cancer (SCLC), gastric cancer, head and neck cancer, brain cancer, renal cancer, lymphoma and leukaemia.

8. The use of the combination according to any one of claims 1-3, in the manufacture of a medicament for use in the production of an anti-angiogenic effect, in a warm-blooded animal, such as man.

9. The use according to claim 7 wherein the cancer is prostate cancer.

10. The use according to claim 7 wherein the cancer is NSCLC.

11. The use according to claim 7 wherein the cancer is in a metastatic state.

12. The use according to claim 7 wherein the cancer is in a non-metastatic state.

13. The use according to claim 7 wherein the cancer is renal, thyroid, lung, breast or prostate cancer that is producing bone metastases.

## Patentansprüche

1. Kombination, enthaltend *N*-(3-Methoxy-5-methylpyrazin-2-yl)-2-(4-[1,3,4-oxadiazol-2-yl]phenyl)-pyridin-3-sulfonamid (ZD4054) oder ein pharmazeutisch annehmbares Salz davon und einen EGFR-TKI oder ein pharmazeutisch annehmbares Salz davon.

2. Kombination nach Anspruch 1, in der der EGFR-TKI ausgewählt ist unter:
*N*-(3-Chlor-4-fluorphenyl)-7-methoxy-6-(3-morpholinopropoxy)chinazolin-4-amin (ZD1839);
*N*-3-(Ethinylphenyl)-6,7-bis-(2-methoxyethoxy)-chinazolin-4-amin oder ein pharmazeutisch annehmbares Salz davon (gekoppelt mit den Code-Nummern CP 358774 und OSI-774 (Monomethansulfonatsalz));
6-Acrylamido-*N*-(3-chlor-4-fluorphenyl)-7-(3-morpholinopropoxy)chinazolin-4-amin (gekoppelt mit den Code-Nummern PD 183805 und CI 1033);
4-[(1R)-1-Phenylethylamino]-6-(4-hydroxyphenyl)-7H-pyrrolo[2,3-*d*]pyrimidin (gekoppelt mit den Code-Nummern PKI-166, CGP 75166 und CGP 59326);
*N*-[4-(3-Bromanilino)chinazolin-6-yl]but-2-inamid (gekoppelt mit den Code-Nummern CL-387785 und EKB-785) und
4-(3-Chlor-4-fluoranilino)-3-cyano-6-(4-dimethyl-aminobut-2(E)-enamido)-7-ethoxychinolin (EKB-569);
oder einem pharmazeutisch annehmbaren Salz davon.

3. Kombination nach Anspruch 1 oder 2, in der der EGFR-TKI unter ZD1839 oder einem pharmazeutisch annehmbaren Salz davon ausgewählt ist.

4. Kombination nach einem der Ansprüche 1-3 zur Verwendung als Arzneimittel.

5. Pharmazeutische Zusammensetzung, enthaltend eine Kombination nach einem der Ansprüche 1-3 zusammen mit einem pharmazeutisch annehmbaren Verdünnungsmittel oder Träger.

6. Verwendung einer Kombination nach einem der Ansprüche 1-3 bei der Herstellung eines Arzneimittels zur Behandlung von Krebs bei einem Warmblüter wie dem Menschen.

7. Verwendung nach Anspruch 6, bei der es sich bei dem Krebs um Speiseröhrenkrebs, Myelom, Leberzellkrebs, Bauchspeicheldrüsenkrebs, Zervixkrebs, Ewing-Sarkom, Neuroblastom, Kaposi-Sarkom, Eierstockkrebs, Brustkrebs, Kolorektalkrebs, Prostatakrebs, Blasenkrebs, Melanom, Lungenkrebs - nicht-kleinzelligen Lungenkrebs (NSCLC) und kleinzelligen Lungenkrebs (SCLC), Magenkrebs, Kopf- und Halskrebs, Hirnkrebs, Nierenkrebs, Lymphom und Leukämie handelt.

8. Verwendung der Kombination nach einem der Ansprüche 1-3 bei der Herstellung eines Arzneimittels zur Verwendung bei der Erzielung einer antiangiogenen Wirkung bei einem Warmblüter wie dem Menschen.

9. Verwendung nach Anspruch 7, bei der es sich bei dem Krebs um Prostatakrebs handelt.

10. Verwendung nach Anspruch 7, bei der es sich bei dem Krebs um NSCLC handelt.

11. Verwendung nach Anspruch 7, bei der sich der Krebs in einem metastasierenden Stadium befindet.

12. Verwendung nach Anspruch 7, bei der sich der Krebs in einem nicht metastasierenden Stadium befindet.

13. Verwendung nach Anspruch 7, bei der es sich bei dem Krebs um Knochenmetastasen produzierenden Nieren-, Schilddrüsen-, Lungen-, Brust- oder Prostatakrebs handelt.

## Revendications

1. Combinaison comprenant le *N*-(3-méthoxy-5-méthylpyrazin-2-yl)-2-(4-[1,3,4-oxadiazol-2-yl]phényl)pyridine-3-sulfonamide (ZD4054), ou un sel de qualité pharmaceutique de ce composé, et un EGFR-TKI ou un sel de qualité pharmaceutique de ce composé.

2. Combinaison selon la revendication 1 où le EGFR TKI est sélectionné parmi :
la *N*-(3-chloro-4-fluorophényl)-7-méthoxy-6-(3-morpholinopropoxy)quinazoline-4-amine (zD1839); la *N-*(3-éthynylphényl)-6,7-bis(2-méthoxyéthoxy)quinazoline-4-amine, ou un sel de qualité pharmaceutique dudit composé (lié aux codes CP 358774 et OSI-774 (sel de monométhanesulfonate)) ;
la 6-acrylamido-*N*-(3-chloro-4-fluorophényl)-7-(3-morpholinopropoxy)quinazoline-4-amine (liée aux codes PD 183805 et CI 1033) ;
la 4-[(*1R*)-1-phényléthylamino]-6-(4-hydroxyphényl)-7H-pyrrolo[2,3-*d*]pyrimidine (liée aux codes PKI-166, CGP 75166 et CGP 59326) ;
le *N*-[4-(3-bromoanilino)quinazoline-6-yl]but-2-ynamide (lié aux codes CL-387785 et EKB-785) ;
et la 4-(3-chloro-4-fluoroanilino)-3-cyano-6-(4-diméthylaminobut-2(*E*)-énamido)-7-éthoxyquinoline (EKB-569) ;
ou un sel de qualité pharmaceutique de l'un de ces composés.

3. Combinaison selon la revendication 1 ou 2 où le EGFR-TKI est sélectionné parmi ZD1839 et les sels de qualité pharmaceutique de ce composé.

4. Combinaison selon l'une quelconque des revendications 1-3 pour emploi en tant que médicament.

5. Composition pharmaceutique comprenant une combinaison selon l'une quelconque des revendications 1-3, associée à un diluant ou un vecteur de qualité pharmaceutique.

6. Emploi d'une combinaison selon l'une quelconque des revendications 1-3 dans la fabrication d'un médicament pouvant être employé dans le traitement de cancers chez un animal à sang chaud tel que l'homme.

7. Emploi selon la revendication 6 où le cancer est un cancer de l'oesophage, un myélome, un cancer hépatocellulaire, pancréatique ou cervical, une tumeur d'Ewings, un neuroblastome, un sarcome de Kaposis, un cancer de l'ovaire, un cancer du sein, un cancer colorectal, un cancer de la prostate, un cancer de la vessie, un mélanome, un cancer bronchique - cancer bronchique non à petites cellules (CBNPC) et cancer bronchique à petites cellules (CBPC), un cancer gastrique, un cancer de la tête et du cou, un cancer du cerveau, un cancer rénal, un lymphome ou une leucémie.

8. Emploi de la combinaison selon l'une quelconque des revendications 1-3 dans la fabrication d'un médicament pouvant être employé dans la production d'un effet anti-angiogénique, chez un animal à sang chaud tel que l'homme.

9. Emploi selon la revendication 7 où le cancer est un cancer de la prostate.

10. Emploi selon la revendication 7 où le cancer est un CBNPC.

11. Emploi selon la revendication 7 où le cancer est dans un état métastatique.

12. Emploi selon la revendication 7 où le cancer est dans un état non-métastatique.

13. Emploi selon la revendication 7 où le cancer est un cancer rénal, thyroïdien, bronchique, du sein ou de la prostate qui produit des métastases osseuses.
